# FASCICULE DE BREVET EUROPEEN

(11) **EP 1 522 286 B1**
(45) Date de publication et mention de la délivrance du brevet: **12.01.2011**
(21) Numéro de dépôt: 04292007.4
(22) Date de dépôt: 06.08.2004
(51) Int. Cl.: A61F 2/58

(54) **Dispositif mécanique permettant la variation des angulations des interphalanges proximales dans les prothèses de doigts et dans les doigts de prothèses de mains**
Mechanische Vorrichtung zur Winkelveränderung der proximalen Jnterphalangealenin Fingerprothesen und in den Fingern von Handprothesen.
Mechanieal device for varying the angles of proximal interphalangeals in the fingers prothesis and in fingers of hand prothesis.

(30) Priorité: 14.08.2003 FR 0309938
(43) Date de publication de la demande: 13.04.2005
(73) Titulaire: Paronuzzi-Feltrin, Noemi, 02310 Nogent l'Artaud (FR); Feltrin, Oscar, 47822 Santarcangelo di Romana (RN) (IT); Bernhardt-feltrin, Adeline, 47822 Santarcangelo di Romana (RN) (IT)
(72) Inventeur: Feltrin, Oscar, 47822 Santarcangelo di Romagna (RN) (IT); Bernhardt-Feltrin, Adeline, 47822 Santarcangelo di Romana (RN) (IT)

(56) Documents cités:
- DE-C- 317 183
- FR-A- 581 115
- FR-A- 606 940
- GB-A- 650 501
- US-A- 2 598 593

## Description

La présente invention a trait d'une manière générale à un dispositif aux dimensions inférieures à un doigt à insérer dans les prothèses de mains et de doigts selon le préambule de la revendication 1.

De nombreux amputés de doigts, de mains partielles et de mains totales ressentent le besoin d'avoir en plus de l'esthétique une fonctionnalité qui compléterait ainsi une apparence quasi normale pour satisfaire les besoins psychologiques et pratiques dans leur vie quotidienne. Pouvoir positionner simplement et rapidement un ou plusieurs doigts afin d'améliorer et assurer la préhension d'un objet, est pour le patient un supplément primordial.

Dans l'état actuel de la technique des tentatives sont décrites dans le brevet français FR-2557450 qui est relatif uniquement aux prothèses de mains totales dont le fonctionnement se fait par tirage de câbles et dont chaque doigt ne fonctionne qu'avec l'ensemble de la main.

Suivant un autre procédé de perfectionnement apporté par le brevet français FR-2665833 destiné aux prothèses totales de la main concerne le verrouillage des doigts en position fermée par système de câble faisant traction simultanée sur quatre doigts

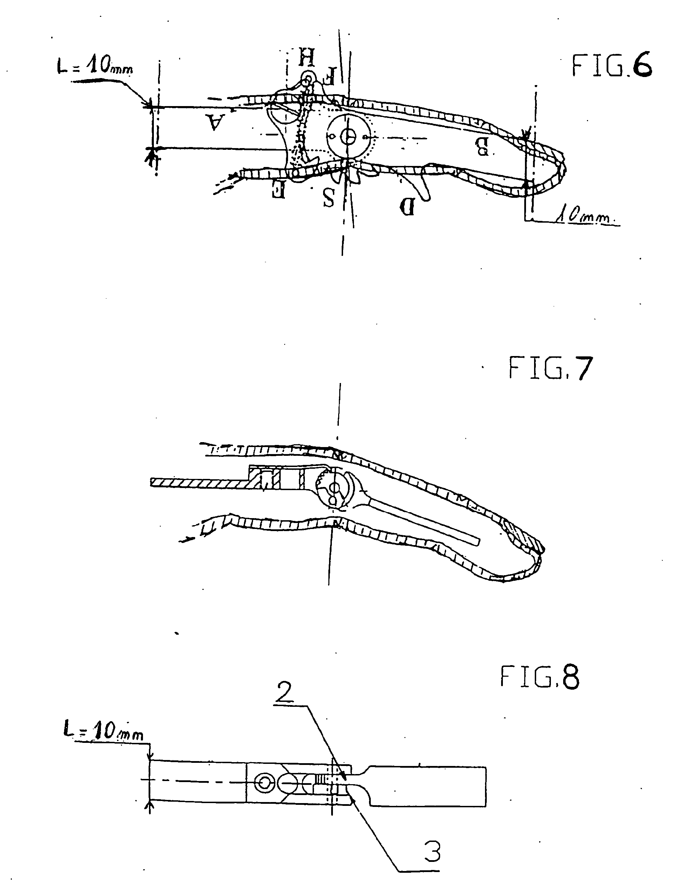

Suivant un autre dispositif décrit dans le brevet français FR-581.115 délivré le 19 septembre 1924, il apparait qu'aux dimensions réelles d'un doigt, comme représenté sur la Fig. 6, du fait des proéminences de la came F, de l'ergot H, du cliquet E, de la denture S et de la came D ce dispositif n'est pas insérable dans un doigt prothétique.

La présente invention a pour but de proposer un dispositif aux dimensions insérables dans les prothèses de mains et de doigts selon les caractéristiques de la revendication 1. Le dispositif a la possibilité de faire varier l'angulation des interphalanges proximales de la prothèse par un actionnement manuel et de rester dans la position désirée à l'aide de crans réalisés dans l'articulation du dispositif comme il sera décrit successivement, et de retourner dans la position initiale.
La figure 1 représente en coupe le système dans la position d'angulation minimum de départ.
La figure 2, la vue de dessus.
La figure 3, la coupe avec l'angulation en variation maximum.

Les dispositifs selon l'invention sont obtenus par la fabrication et l'assemblage de pièces usinées (1-2-3-4-5-6-7), (8) étant une partie plate.

Le système est composé des pièces suivantes:
- support à fourche (1)
- tige plate avec crans de positionnement (2)
- came (3)
ressort de positionnement à lame (4)
- axe de rotation (5)
- picot d'entraînement de came (6)
- vis de fixation de ressort (7)
Tel dispositif comprend un élément de support (1), à forme de fourche, dans lequel vient s'insérer la tige plate crantée (2) et la came (3), pivotant sur l'axe (5), de façon à permettre une rotation par rapport au support (1).

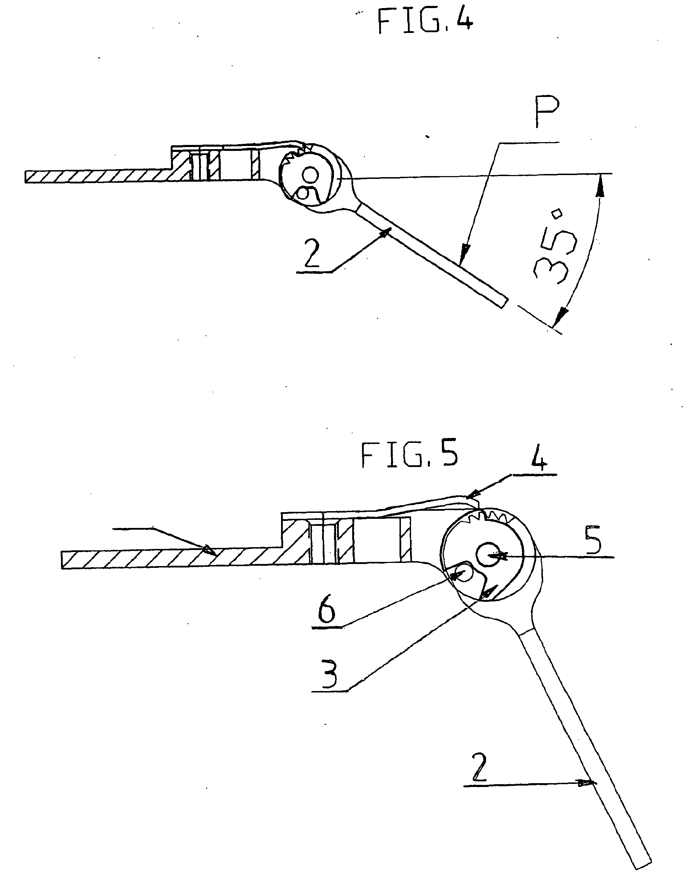

Sur la partie supérieure du support (1), est positionné un ressort à lame plat (4), fixé avec la vis (7), la partie finale du ressort plat (4), correctement façonnée, s'insert dans les crans de la tige plate (2) de façon à en déterminer la position.
Le ressort (4) est positionné de façon à permettre une rotation et la variation de l'angulation par rapport au support (1), avec une légère pression sur la tige plate (2) comme representé sur la Fig. 4, de façon à enclencher le ressort (4) dans le cran successif de l'articulation (2).
La position du ressort (4) ne permet pas la rotation de la tige plate (2) en sens inverse permettant ainsi d'obtenir une forte prise avec l'objet.

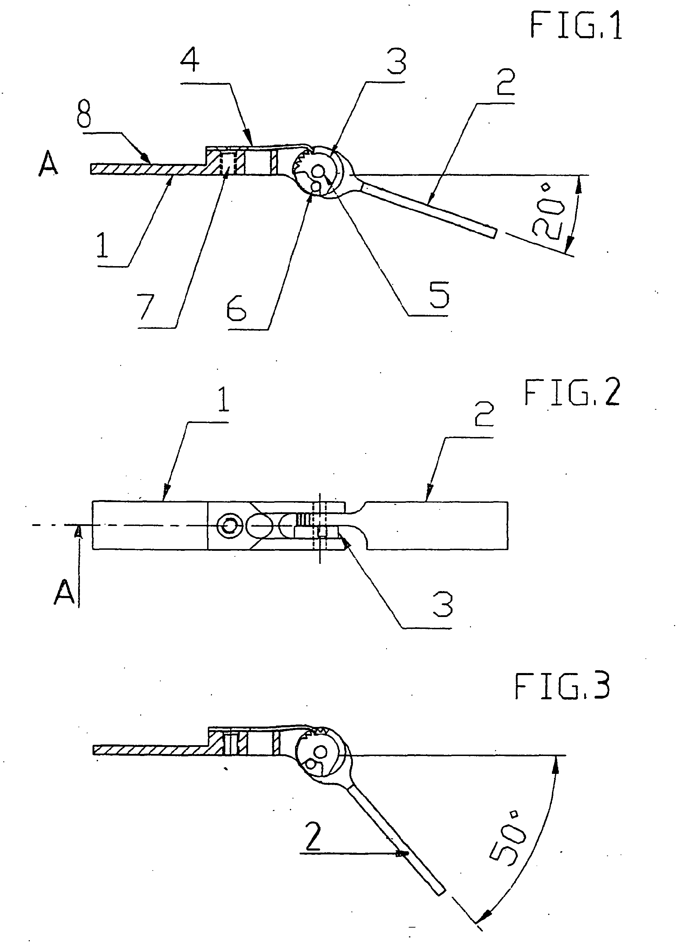

Aprés avoir atteint l'angulation maximum, ici représentée par la Fig.3 à 50° en faisant faire à la tige plate (2) Fig.5 une rotation d'environ 15°-20° de plus, le picot d'entraînement (6), entraîne en rotation la came (3), soulevant le ressort (4), de façon à libérer les crans de la tige plate (2) comme représenté dans la Fig. 5. Le ressort (4) étant soulevé, la rotation de la tige plate (2) en sens inverse est possible.

En exécutant la rotation de la tige plate (2) en sens contraire, en atteignant donc l'angulation initiale Fig. 1, le picot d'entrainement (6) fait tourner la came (3), libérant le ressort (4), lequel s'insert automatiquement dans le premier cran de la tige plate (2) recréant ainsi la situation initiale avec l'angulation minimum de départ.

Dans ce dispositif il est possible de faire varier l'angulation initiale Fig.5 en intervenant sur la position des crans de la tige plate (2) et sur le picot d'entraînement (6), monté sur la tige plate (2).

Dans ce dispositif il est possible de faire varier l'angulation maximum fig. 3 en modifiant la forme de la came (3), ce qui permet de retarder ou anticiper le soulèvement du ressort (4). Dans la Fig. 1, la came est représentée à titre d'exemple avec une angulation maximale différente des autres dessins. On obtient ainsi différents dispositifs:
1°) un dispositif permettant la modification de l'angulation des doigts dans une prothèse de doigt, de main totale ou de main partielle.
2°) un dispositif à came (3) qui en fin de course, donc après avoir atteint l'angulation maximum, libère le blocage de l'angulation et permet à la prothèse de retroumer automatiquement à sa position initiale, ceci étant dû soit à la mémoire de forme des matériaux utilisés pour la prothèse, mousse polyurethane ou silicone soit, si le matériau utilisé n'a pas de mémoire de forme, la remise en position manuellement par le patient lui-même.
3°) un dispositif permettant de régler l'angulation minimum de départ, l'angulation maximum et les angulations intermédiaires en remplaçant la tige plate (2), Fig. 1, par une tige plate ayant une disposition et un nombre de crans différents.
4°) un dispositif qui à l'aide du ressort plat (4) Fig.1 permet le blocage de l'articulation (2) quelle que soit l'angulation choisie ce qui permet d'apporter une force d'oppositon afin de permettre le blocage des objets dans la prothèse.

Bien entendu on pourra adopter differents types de matériaux, méthodes d'usinage, formes ou varier la dimension de ces pièces à assembler sans pour autant sortir du cadre de l'invention.

## Revendications

1. Dispositif mécanique permettant de régler différents angles de la main ou du doigt avec blocage en position, pouvant être intégré dans des prothèses passives de doigt ou de main, **caractérisé en ce qu'**il comprend un élément de support (1) en forme de fourche, dans lequel vient s'insérer une tige crantée (2) et une came (3), les deux pivotant sur un axe (5), de façon à permettre une rotation par rapport au support (1) entre un angle minimum et un angle maximum, sur la partie supérieure dudit support (1), est positionné un ressort à lame plate (4) fixé avec une vis (7) dont une extrémité de la lame s'insère dans les crans de la tige plate (2) pour déterminer la position ou l'angle de la tige par rapport au support, le dispositif comprend en outre un picot d'entraînement (6) destiné à coopérer avec la came de telle sorte que lorsque l'angle maximum est atteint, par une rotation supplémentaire d'environ 15°-20° de la tige, ledit picot entraîne en rotation la came, soulevant ainsi le ressort (4) de façon à libérer les crans de la tige plate et permettant ainsi la rotation de la tige plate en sensé inverse.

2. Dispositif selon la revendication 1, **caractérisé en ce que** la came (3) qui en fin de course, après avoir atteint l'angle maximum, libère le blocage de l'angulation et permet à la prothèse de retourner automatiquement à sa position initiale grâce à la mémoire de forme des matériaux utilisés pour la prothèse, mousse polyuréthane ou silicone, ou manuellement par le patient lui-même.

3. Dispositif selon l'une quelconque des revendications précédentes **caractérisé en ce qu'**il permet de régler l'angle minimum de départ, l'angle maximum et les angles intermédiaires en ramplaçant la tige plate (2) par une tige plate ayant une disposition et un nombre de crans différents.

## Claims

1. Mechanical device, which allows adjusting different angles of hand or fingers blocking them in position, designed to supplement passive prosthesis of fingers or hand, **characterised by** a fork-shaped support (1), in which a shaft with notches (2) and a cam (3) are inserted. These two elements turning on an axis (5) allow a rotation in relation to a support (1) between a minimum and a maximum angle. On the upper part of the above-mentioned support (1), a flat spring (4) is positioned fixed with a screw (7) whose extremity is inserted in the notches of the flat shaft (2) so as to determine the position or the angle of the shaft in relation to the support. The device consists also of a rotation wedge (6) designed to cooperate with the cam so that, when the maximum angle is reached, through an additional rotation of about 15°-20° of the shaft, the above-mentioned wedge drags the cam into the rotation, lifting thereby the spring (4) in order to release the notches of the flat shaft, thus allowing the rotation of the shaft in the opposite direction.

2. A device as per claim 1, **characterised by** a cam (3) that in the limit position, namely after having reached the maximum angle, releases the blocking of the angle and allows the prosthesis to return automatically, through the memory of the shapes of the material used for the prosthesis, polyurethane resin or silicone, or manually by the patient, to the initial position.

3. A device as per any previous claim, **characterised by** the possibility of giving the minimum starting angle, the maximum angle and the intermediate angles by replacing the flat shaft (2) with another shaft having a different positioning and number of notches.

## Patentansprüche

1. Mechanische Vorrichtung, die die verschiedenen Winkel der Hand oder der Finger reguliert und die Stellungen blockiert, eingegliedert in der Prothese von passiven Fingern oder Händen, charakterisiert durch ein Zusatzteil (1) in Form einer Gabel, in der ein Schaft mit Kerben (2) und eine Nockenwelle (3) versehen sind. Diese beiden drehen sich um eine Achse (5), so dass eine Drehung, auf das Zusatzteil (1) bezogen, zwischen dem minimalen und maximalen Winkel möglich ist. Auf der oberen Seite des besagten Zusatzteils (1) ist eine flache Feder (4) mit einer Schraube (7) angebracht. Das Ende der Schraube wird in den Kerben des flachen Schaftes (2) eingegliedert um die Position oder den Winkel des Schaftes auf das Zusatzteil bezogen, zu bestimmen. Diese Vorrichtung besteht außerdem aus einem Antriebskeil (6), der bestimmt ist, mit der Nockenwelle zu kooperieren, so dass, wenn der maximale Winkel durch eine Zusatzdrehung von ca. 15°-20° des Schaftes erreicht ist, der besagte Keil die Nockenwelle in die Drehung zieht und die Feder (4) hebt, so dass die Kerben des flachen Schaftes aufgehoben sind und die Drehung des flachen Schaftes in die 5 umgekehrte Richtung möglich ist.

2. Vorrichtung gemäß Anspruch 1, wobei eine Nockenwelle (3), die in der Endstufe, d.h. nach Erreichen des maximale Winkels, die Blockierung aufhebt und der Prothese erlaubt automatisch, dank der Eigenschaften der Materialformen für Prothesen, Polyurethan Harz oder Silikone, oder manuell beim Patienten selbst, in die Ausgangsposition zurückzukehren.

3. Vorrichtung gemäß jedem vorherigen Anspruch, wobei der minimale Winkel beim Start, maximale Winkel und Zwischenwinkel durch das Austauschen des flachen Schaftes (2) gegen einen anderen mit verschiedenen Anordnungen und Anzahl von Kerben, möglich ist.
